Europäisches Patentamt

European Patent Office

Office européen des brevets

(19)

(11) **EP 0 620 420 B1**

(12) **EUROPEAN PATENT SPECIFICATION**

(45) Date of publication and mention
of the grant of the patent:
**14.09.2005 Bulletin 2005/37**

(51) Int Cl.⁷: **G01F 1/56**, G01P 5/08,
A61B 5/026, A61N 1/36

(21) Application number: **94100130.7**

(22) Date of filing: **05.01.1994**

(54) **Method and device for measuring the flow of an electrolytic fluid**

Verfahren und Anordnung zur Messung des Durchflusses einer elektrolytischen Flüssigkeit

Procédé et dispositif pour mesurer le débit d'un fluide électrolytique

(84) Designated Contracting States:
**DE FR GB IT NL**

(30) Priority: **29.01.1993 SE 9300282**

(43) Date of publication of application:
**19.10.1994 Bulletin 1994/42**

(73) Proprietor: **St. Jude Medical AB**
**175 84 Järfälla (SE)**

(72) Inventor: **Strandberg, Hans**
**S-172 36 Sundbyberg (SE)**

(56) References cited:
**EP-A- 0 049 027        EP-A- 0 077 413**
**EP-A- 0 310 026        US-A- 3 450 984**
**US-A- 5 174 299**

## Description

This invention relates to a method and a device for measuring the flow of an electrolytic fluid, at least two electrodes then being immersed in the electrolyte.

This type of flow measurement has a number of industrial and medical applications, such as the measurement of blood flow for rate-responsive control of a pacemaker and in conjunction with the detection of tachycardia. Blood flow is reduced in tachycardia.

This type of flow measurement has previously been performed by e.g. thermodilution, the cooling of a heated electrode by fluid flowing around it then being studied. The electrode can be heated and the temperature in the medium can be e.g. alternately sensed. Performing such flow measurements with the aid of Doppler sound measurement is also previously known. Both these prior art methods require relatively complex equipment.

The document EP-A-0 077 413 discloses a system for measuring the flow rate of electrolytic fluids including a flow cell with a cylindrical channel for the fluid flow and a pair of spaced apart electrodes conformed to the cylindrical surface of the flow channel. The electrodes are electrically conductive and inert to the fluid and are in contact with the fluid flow for a desired range of flow measurement. A monopolar pulse train is applied across the electrodes to effect a cell impedance which is inversely proportional to the rate of flow. A control signal is produced in response to the potential across the electrodes and this signal is applied to the pulse train generator varying the pulse train current to effect an increase in the current in response to an increase in flow rate whereby the cell impedance is stabilized for every flow rate and the electrode potential is representative of the flow rate.

The document EP-A-0 310 026 discloses a heart pacemaker comprising a pulse generator for generating stimulation pulses, a single sensor with a pacing electrode positioned in a patient's right heart for deriving a signal modulated with a multiplicity of physiological functional parameters related to a patient's condition such as changes in volume, flow or pressure in the patient's vascular system.

The object of the present invention is to achieve a new method and a new device for measuring the flow of an electrolytic fluid which are simpler and require simpler and, thus, cheaper measurement equipment.

This object is achieved with a method and a device of the kind described in the introduction with the characterizing features set forth in claims 1 and 5 respectively.

The method and device according to the invention are based on the circumstance that an EMF develops between electrodes when electrodes made of a suitable material are immersed in an electrolyte, such as a saline solution. This EMF, or more accurately, the terminal voltages arising between the respective electrode and the electrolyte and giving rise to the resulting EMF, depends on the flow of fluid around the electrode.

The advantages of the invention lies in its simplicity. The measurement equipment required is simple, and an existing pacemaker electrode, if of the right type, can be employed when the invention is used for measurement of blood flow for pacemakers. Thus, a bipolar electrode with e.g. a carbon tip and platinum ring, has been found to function very well for such measurement of blood flow according to the invention.

According to the method of the invention, a continuous or recurrent pulsed current, providing a net direct current, is applied to the electrodes, and the resulting voltage between the electrodes, which depends on the flow of fluid, is measured. In this manner, the electrode system is "charged" with an EMF.

After a long period of time, the unloaded EMF amounts to about 40-50 mV. If, in the measurement procedure, the system is pulsed with a few volts whose pulse duration lasts a fraction of 1 ms, an EMF of less than 1 mV arises with a recharge constant of the order of minutes.

According to an alternative embodiment of the method according to the invention, the pulse duration is regulated so the measured voltage becomes appropriate to the application in question.

However, stimulation till a stable, elevated EMF is achieved provides a very good possibility for detecting flow. The flow-dependent variation in EMF amounts to about 50 mV.

Measurement of flow can also be performed by compensatorily adjustment of the pulse amplitude to keep the EMF voltage stable. Experiments have been made at a 0.5 V EMF and a 1 V EMF level. Changes in pulse voltage of 1 - 2 V were then required between a fast fluid flow and a stagnant fluid flow.

According to another advantageous embodiment of the method according to the invention, voltage between the electrodes is kept constant, and the current between the electrodes is measured. Since the charge transferred is a measure of the fluid flow, an increased flow results in an increase in current.

No saturation has been observed. Both a zero flow current and sensitivity, i.e. current/flow rate, depend on the fluid and the electrodes used and the voltage applied. The measurement current is of the order of 50 -500 nA.

When the invention is applied to a pacemaker, it must be kept in mind that the EMF, or terminal voltages, is greatly dependent on the stimulation pulses emitted. However, compensating for this is not very difficult, since the stimulation rate is deterministic and measurable, and measurement of current or voltage is performed immediately prior to the emission of a stimulation pulse according to an advantageous example of the method according to the invention. Studies have shown that the post-polarization course is not dependent on the fluid flow.

The voltage, which can be characterized as electrode EMF, is material-dependent. A reduction, harmless to the electrode material, occurs at the negative electrode. However, an oxidation occurring at the positive electrode can cause dissolution of the material, i.e. the electrode can be eaten away. So the positive electrode must be made of a non-reactive material. Therefore, the choice of electrode material must be made with care, as well as the choice of current direction, for the flow measurement.

According to an advantageous embodiment of the device according to the invention when used with pacemakers, the electrodes consist of a bipolar pacemaker electrode with a carbon tip and a platinum ring. Practical tests with this electrode configuration have yielded excellent flow measurement results.

The invention will now be explained in greater detail, with reference to the attached drawings illustrating embodiments. Thus,

FIG. 1 shows a pair of electrodes in an electrolyte to illustrate the invention's basic principle;

FIG. 2 shows an example of a circuit for performing the voltage measurement required for determining the flow;

FIGS. 3 and 4 show examples of measurement circuits in the device according to the invention;

FIG. 5 schematically depicts a pacemaker or defibrillator with the device according to the invention;

FIG. 6 is a block diagram of a pacemaker or defibrillator with the device according to the invention; and

FIG. 7 is a block diagram of an example of the flow measurement device according to the invention in the version shown in FIG. 6.

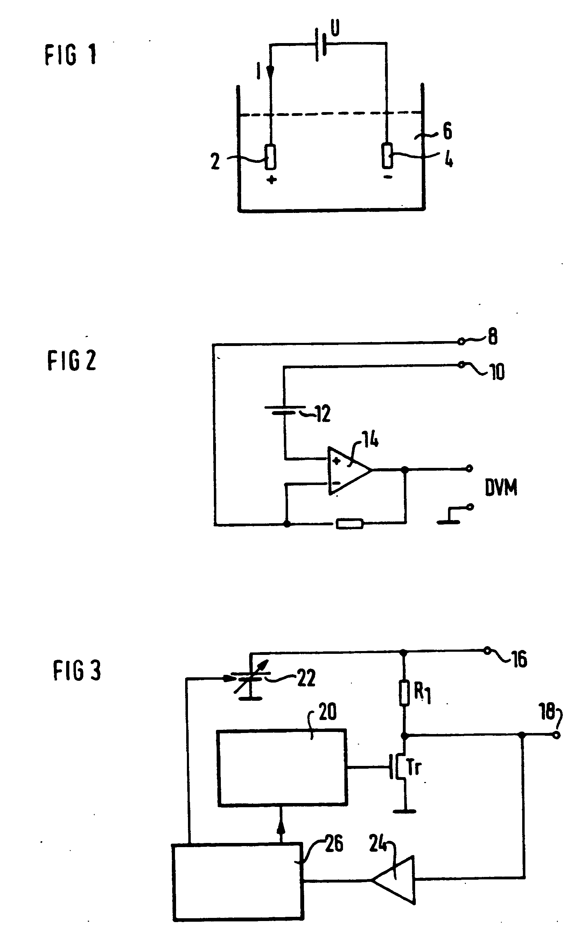

In FIG. 1 is shown two electrodes 2, 4 immersed in an electrolyte 6, e.g. a 0.5% saline solution.

When suitable electrodes are chosen, an EMF, which depends on the flow of electrolyte 6, develops between the electrodes 2, 4. More precisely flow-dependent terminal voltages develop between the electrode 2, 4 and the electrolyte, resulting in the EMF.

In FIG. 1, a voltage U is applied across the electrodes 2, 4, and the current I is intended to be measured and its magnitude constitutes a measure of the flow of the electrolyte.

The principle of the invention can be illustrated with the device shown in FIG. 1 by stirring the electrolyte 6 with e.g. a magnetic stirrer in order to provide a flow in the electrolyte 6 container.

The current I through the pair of electrodes (regarded as corresponding voltage) can be measured with the circuit shown in FIG. 2, a suitable recording instrument being connected to DVM. A reference image memory oscilloscope, i.e. an oscilloscope supplied with a normal pulse to which measured pulses can be compared to make deviations readily discernible to the eye, can be used as such a recording instrument.

When the circuit in FIG. 2 is used in a pacemaker with a bipolar electrode, the output terminal 8 is connected to the tip of the stimulating electrode, appropriately a carbon tip, and the output terminal 10 is connected to the sensing electrode, appropriately a platinum ring. The control voltage is supplied by the voltage source 12, and the voltage between output terminals 8 and 10 is measured with the feedback amplifier 14.

A lack of dissolved oxygen in the electrolyte has been found to produce unstable, non-reproducible results. So oxygen must be present dissolved in the electrolyte if flow measurement according to the invention shall operate reliably. Moreover, the pH value of the electrolyte has been found to affect the sensitivity of flow measurement. The limit value for reliable measurement with the method and device according to the invention is a pH of about 5, a more acidic solution impairing sensitivity. The processes at the two electrodes are:

$$2OH^- \rightarrow H_2O + \tfrac{1}{2}O_2 + 2e^- \qquad \text{oxidation}$$

$$H_2O + \tfrac{1}{2}O_2 + 2e^- \rightarrow 2OH^- \qquad \text{reduction}$$

With a constant voltage between the two electrodes, any change in the normal potential of an electrode will also cause a change in the normal potential of the other electrode. The sensitivity to voltage changes of the oxidation process above increases as the solution becomes more acidic due to the lower concentration of hydroxide ions.

Experiments have also shown that the flow sensing primarily occurs at the positive electrode, whereas the negative electrode controls the current.

In FIG. 3 is shown a version of the device according to the invention, intended for use in a pacemaker, for the measurement of blood flow. This embodiment is intended for pulsed or sampled flow measurement.

In the circuit according to FIG. 3, the output terminal 16 is connected to the indifferent electrode, suitably a platinum ring, and the output terminal 18 to the stimulation electrode, suitably a carbon tip, cf. the description of FIG. 2.

A high-resistance resistor $R_1$ is connected between the output terminals 16, 18, and the electrode connected to the output terminal 18 is charged with charging pulses by the switching transistor Tr. Thus, these pulses are emitted by the same electrode as the pacemaker's stimulation pulses but at different times.

The switching transistor Tr is controlled by the control electronics 20.

An FET amplifier 24 and the following measurement and control electronics 26 are connected to the output terminal 18 to control the voltage source 22 and for the measurement. The measurement and control electronics 26 are further connected to the transistor's Tr control electronics 20. In this way control of both the transistor Tr and the voltage source 22 is possible depending on the voltage measured.

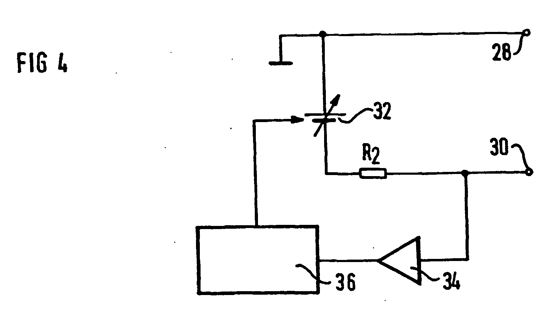

In FIG. 4 is shown an embodiment intended for continuous supply of current to the electrodes. Here, the indifferent electrode is connected to the output terminal 28 and the stimulation electrode to the output terminal 30.

The source of voltage 32, connected in series with a high-resistance resistor $R_2$, is connected between the output terminals 28, 30.

In the corresponding manner as in the version in FIG. 3, an FET amplifier 34 and following measurement and control electronics 36 are connected to the output terminal 30 for measurement and control purposes. Also in this instance, the source of voltage 32 is controllable, depending on the voltage measured between the electrodes.

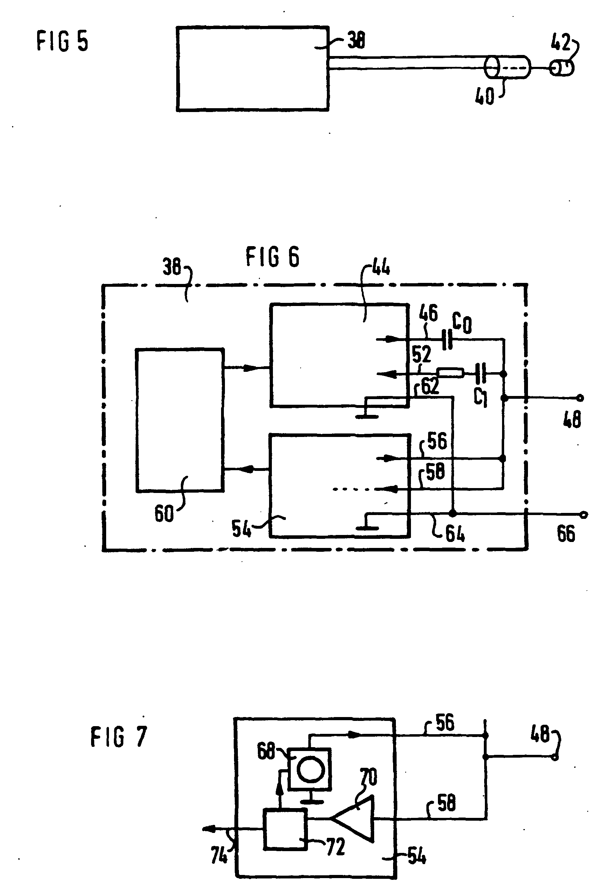

In FIG. 5 is shown a pacemaker or defibrillator 38 with a conventional bipolar electrode with an indifferent ring 40 and a stimulation pole 42. The pacemaker or defibrillator 38 is equipped with a device according to the invention, and the ring 40 for this purpose is appropriately made of platinum and the pole 42 of carbon.

Practical tests have been made with a platinum ring with a contact area of about 32 mm$^2$ and a carbon tip with a 6 mm$^2$ contact area.

In FIG. 6 is shown a block diagram of the pacemaker or defibrillator 38 of FIG. 5.

The pacemaker section 44 is connected with its stimulation output terminal 46, via an output capacitor $C_0$, to the output terminal 48 for the stimulation electrode. Heart signals are received at the same output terminal 48 and supplied via a filter 50 with a capacitor $C_1$ to the pacemaker section 44 via its input 52 for heart signal detection.

The pacemaker or defibrillator 38 further contains a flow measurement unit 54 with an output 56 connected to the output 48 in order to supply the stimulation electrode with a bias signal in conjunction with the flow measurement. The voltage measurement signal required for measurement of the flow is received by the flow measurement unit via the input terminal 58.

Special control electronics 60 are provided to control the pacemaker section 44, in response to the flow measurement unit 54.

Both the pacemaker section 44 and the flow measurement unit 54 are further connected, via outputs 62, 64, to the output 66, intended to be connected to the indifferent electrode 40 in FIG. 5.

In FIG. 7 is shown the flow measurement unit 54 in greater detail. Thus, the flow measurement unit 54 contains signal-generating electronics 68 for supplying, via the output 56 and the output 48, the electrode with currents or voltages required for measurement of the flow, as discussed above. The measurement signal for the flow measurement is received via the output 48 and the input 58 and sent via a buffer amplifier 70 to the measurement and control electronics 72 which treat and analyze the measurement signal in an appropriate manner and deliver an output signal on the output 74 representing the measured flow value. The measurement and control electronics 72 are also connected to the signal-generating electronics 68 to control their function, in response to the measurement signals received.

## Claims

1. A method of measuring the flow of an electrolytic fluid (6), in which the current is measured between two stationary electrodes (2,4; 40, 42) immersed in said electrolytic fluid, **characterised in that** electrochemical reactions are caused at each stationary electrode in said electrolytic fluid, by supplying a continuous or pulsed voltage to said two stationary electrodes (2, 4; 40, 42), such that an EMF is developed between said electrodes which depends on the flow of fluid around the electrodes, **in that** the current between said electrodes is measured and the supplied voltage is kept constant during the measurement, the measurement current being of the order of 50-500 nA and being flow dependent.

2. A method of measuring the flow of an electrolytic fluid (6) in which the voltage is measured between two stationary electrodes (2,4; 40,42) immersed in said electrolytic fluid, **characterised in that** electrochemical reactions are caused at each stationary electrode in said electrolytic fluid, by supplying a continuous or pulsed current providing a met direct current to said two stationary electrodes (2, 4; 40, 42), such that an EMF is developed between said electrodes which depends on the flow of fluid around the electrodes and **in that** the voltage between said electrodes is measured the flow dependent variation of the voltage amounting to about 50 mV.

3. Use of the method according to claim 1 or 2 for measuring blood flow in an implantable pacemaker or a defibrillator, which is adapted to deliver stimulation pulses.

4. Use of the method according to claim 1 or 2 for measuring blood flow in an implantable pacemaker or a defibrillator, which is adapted to deliver stimulation pulses **characterised in that** the measurement of the current or voltage is performed immediately prior to the emission of a stimulation pulse.

**5.** A device for measuring the flow of an electrolytic fluid containing at least two outputs (8, 10, 16, 18; 28, 30), said outputs being connectable to electrodes to be stationary immersed in an electrolytic fluid, the flow of which is to be measured, a power source (12, 22, 32) having opposite poles to which said two outputs are respectively connected, for producing respective electrochemical reactions at respective stationary connectable electrode in said electrolytic fluid, such that an EMF is developed between said electrodes which depends on the flow of fluid around the electrodes, said power source supplying a pulsed or continuous current providing a net direct current resulting in a measurement voltage, the flow-dependent variation thereof amounting to about 50 mV, or a pulsed or continuous voltage giving a flow-dependent measurement current of the order of 50-500 nA, a measurement unit (14; 24; 26; 34; 36; 54) for measuring the voltage or the current between said outputs (8, 10; 16, 18; 28, 30), when said connectable electrodes are immersed in the fluid and connected to said outputs(8, 10; 16, 18; 28, 30).

**6.** A pacemaker or defibrillator comprising a device according to claim 5, **characterised in that** said electrodes (2, 4; 40, 42) are electrodes of the implantable pacemaker or defibrillator and **in that** said pacemaker or defibrillator is adapted to make the measurements of the flow-dependent current or flow dependent voltage prior to the emission of a stimulation pulse.

**7.** A pacemaker or defibrillator according to claim 6, **characterised in that** the positive electrode is made from non-reactive material.

**8.** A pacemaker or defibrillator according to claim 6 or 7 **characterised in that** said electrodes (2, 4; 40, 42) consist of a bipolar pacemaker electrode with a carbon tip as the stimulating electrode and a platinum ring as the indifferent electrode.

**9.** A pacemaker according to any of the claims 6 to 8 which has a pacemaker section (44), **characterised in that** control circuits (60) are provided for controlling the pacemaker section (44) in response to the measurement unit (54).

**Patentansprüche**

**1.** Verfahren der Messung des Flusses eines elektrolytischen Fluids (6), bei dem zwischen zwei, in das genannte elektrolytische Fluid eingetauchten, stationären Elektroden (2, 4; 40, 42) der Strom gemessen wird, **dadurch gekennzeichnet, dass** an jeder stationären Elektrode in dem genannten elektrolytischen Fluid durch Anlegen einer kontinuierlichen oder einer impulsförmigen Spannung an die beiden genannten stationären Elektroden (2, 4; 40, 42) elektrochemische Reaktionen verursacht werden, derart, dass zwischen den genannten Elektroden eine elektromotorische Kraft entwickelt wird, die von dem Fluss des Fluids um die Elektroden abhängig ist, dass der Strom zwischen den genannten Elektroden gemessen wird und während der Messung die angelegte Spannung konstant gehalten wird, wobei der Messstrom in der Größenordnung von 50 bis 500 nA liegt und vom Fluss abhängig ist.

**2.** Verfahren des Messens des Flusses eines elektrolytischen Fluids (6), bei dem zwischen zwei, in das genannte elektrolytische Fluid eingetauchten, stationären Elektroden (2, 4; 40, 42) die Spannung gemessen wird, **dadurch gekennzeichnet, dass** an jeder stationären Elektrode in dem genannten elektrolytischen Fluid elektrochemische Reaktionen verursacht werden, durch Zuführen eines kontinuierlichen oder impulsförmigen Stromes, der an die beiden genannten stationären Elektroden (2, 4; 40, 42) einen Nettogleichstrom liefert, derart, dass zwischen den genannten Elektroden eine elektromotorische Kraft entwickelt wird, die von dem Fluss des Fluids um die Elektroden abhängt und dass die Spannung zwischen den genannten Elektroden gemessen wird, wobei die vom Fluss abhängige Veränderung der Spannung sich auf etwa 50 mV beläuft.

**3.** Verwendung des Verfahrens nach Anspruch 1 oder 2 zum Messen des Blutflusses durch einen implantierbaren Schrittmacher oder Defibrillator, der ausgelegt ist, Stimulationsimpulse zu liefern.

**4.** Verwendung des Verfahrens nach Anspruch 1 oder 2 zum Messen des Blutflusses durch einen implantierbaren Schrittmacher oder Defibrillator, der ausgelegt ist, Stimulationsimpulse zu liefern, **dadurch gekennzeichnet, dass** die Messung des Stromes oder der Spannung unmittelbar vor der Ausgabe eines Stimulationsimpulses durchgeführt wird.

**5.** Vorrichtung zum Messen des Flusses eines elektrolytischen Fluids, enthaltend wenigstens zwei Ausgänge (8, 10; 16, 18; 28, 30), wobei die Ausgänge mit Elektroden verbindbar sind, die stationär in ein elektrolytisches Fluid einzutauchen sind, dessen Fluss gemessen werden soll, eine Energiequelle (12, 22, 32) mit Gegenpolen, mit denen die beiden genannten Ausgänge jeweils verbunden sind, zum Erzeugen entsprechender elektrochemischer Reaktionen an der betreffenden stationären, verbindbaren Elektrode in dem genannten elektrolytischen Fluid, derart, dass zwischen den Elektroden eine elektromotorische Kraft entwickelt wird, die von dem Fluss des Fluids um die Elektroden abhängt, dass die genannte Energiequelle einen impulsförmigen oder kontinuierlichen Strom zuführt, der einen Nettogleichstrom liefert, welcher zu einer Messspannung führt, deren flussabhängige Änderung sich auf etwa 50 mV beläuft, oder eine impulsförmige oder kontinuierliche Spannung liefert, die einen flussabhängigen Messstrom in der Größenordnung von 50 bis 500 nA ergibt, eine Messeinheit (14; 24; 26; 24; 36; 54) zum Messen der Spannung an oder des Stromes zwischen den genannten Ausgängen (8, 10; 16, 18; 28, 30), wenn die genannten verbindbaren Elektroden in das Fluid eingetaucht und mit den genannten Ausgängen (8, 10; 16, 18; 28, 30) verbunden sind.

6. Schrittmacher oder Defibrillator, enthaltend eine Vorrichtung nach Anspruch 5, **dadurch gekennzeichnet, dass** die genannten Elektroden (2, 4; 40, 42) Elektroden des implantierbaren Schrittmachers oder Defibrillators sind und dass der genannte Schrittmacher oder Defibrillator ausgelegt ist, die Messungen des vom Fluss abhängigen Stromes oder der vom Fluss abhängigen Spannung vor der Ausgabe eines Stimulationsimpulses durchzuführen.

7. Schrittmacher oder Defibrillator nach Anspruch 6, **dadurch gekennzeichnet, dass** die positive Elektrode aus einem nicht reaktiven Material hergestellt ist.

8. Schrittmacher oder Defibrillator nach Anspruch 6 oder 7, **dadurch gekennzeichnet, dass** die genannten Elektroden (2, 4; 40, 42) aus einer bipolaren Schrittmacherelektrode mit einer Karbonspitze als stimulierender Elektrode und einem Platinring als indifferenter Elektrode gebildet sind.

9. Schrittmacher nach einem der Ansprüche 6 bis 8, der einen Schrittmacherabschnitt (44) enthält, **dadurch gekennzeichnet, dass** eine Steuerschaltung (60) zum Steuern des Schrittmacherabschnittes (44) abhängig von der Messeinheit (54) vorgesehen ist.

**Revendications**

1. Procédé de mesure du débit d'un fluide (6) électrolytique, dans lequel on mesure le courant entre deux électrodes (2, 4, 40, 42) fixes, immergées dans le fluide électrolytique, **caractérisé en ce que** l'on fait se produire des réactions électrochimiques à chaque électrode fixe dans le fluide électrolytique en envoyant une tension continue ou pulsée aux deux électrodes (2, 4, 40, 42) fixes de façon à faire apparaître une FEM entre les électrodes qui dépend du débit du fluide autour des électrodes, on mesure le courant entre les électrodes et on maintient constante la tension appliquée pendant la mesure, le courant mesuré étant de l'ordre de 50 à 500 nA et dépendant du débit.

2. Procédé de mesure du débit d'un fluide (6) électrolytique, dans lequel on mesure la tension entre deux électrodes (2, 4, 40, 42) fixes, immergées dans le fluide électrolytique, **caractérisé en ce que** l'on fait se produire des réactions électrochimiques à chaque électrode fixe dans le fluide électrolytique en fournissant un courant continu ou pulsé donnant un courant continu net aux deux électrodes (2, 4, 40, 42) fixes de façon à faire apparaître une FEM entre les électrodes qui dépend du débit de fluide autour des électrodes et on mesure la tension entre les électrodes, la variation de la tension, qui dépend du débit, s'élevant à environ 50 mV.

3. Utilisation du procédé suivant la revendication 1 ou 2 pour mesurer un débit sanguin dans un stimulateur cardiaque implantable ou un défibrillateur implantable qui est apte à envoyer des impulsions de stimulation.

4. Utilisation du procédé suivant la revendication 1 ou 2 pour mesurer un débit sanguin dans un stimulateur implantable ou dans un défibrillateur implantable qui est apte à envoyer des impulsions de stimulation, **caractérisée en ce que** la mesure du courant ou de la tension est effectuée immédiatement avant l'émission d'une impulsion de stimulation.

5. Dispositif de mesure du débit d'un fluide électrolytique contenant au moins deux bornes (8, 10, 16, 18, 28, 30) de sortie, ces bornes de sortie pouvant être connectées à des électrodes à immerger de manière fixe dans un fluide électrolytique dont le débit doit être mesuré, une source (12, 22, 32) de courant ayant des pôles opposés auxquels les deux bornes de sortie sont connectées, respectivement, pour produire des réactions électrochimiques respectives à l'électrode fixe respective pouvant être connectée dans le fluide électrolytique de façon à faire apparaître une FEM entre les électrodes qui dépend du débit de fluide autour des électrodes, la source de courant fournissant un courant pulsé ou continu donnant un courant continu net se traduisant par une tension de mesure dont la variation en fonction du débit s'élève à environ 50 mV ou une tension pulsée ou continue donnant un courant de mesure qui dépend du débit de l'ordre de 50 à 500 nA, une unité (14, 24, 26, 34, 36, 54) de mesure de la tension ou du courant entre les bornes (8, 10, 16, 18, 28, 30) de sortie lorsque les électrodes pouvant être connectées sont immergées dans le fluide et connectées aux bornes (8, 10, 16, 18, 28, 30) de sortie.

**6.** Stimulateur cardiaque ou défibrillateur comprenant un dispositif suivant la revendication 5, **caractérisé en ce que** les électrodes (2, 4, 40, 42) sont des électrodes du stimulateur cardiaque implantable ou du défibrillateur implantable et **en ce que** le stimulateur cardiaque ou le défibrillateur est conçu pour effectuer les mesures du courant qui dépend du débit ou de la tension qui dépend du débit avant l'émission d'une impulsion de stimulation.

**7.** Stimulateur cardiaque ou défibrillateur suivant la revendication 6, **caractérisé en ce que** l'électrode positive est en un matériau non réactif.

**8.** Stimulateur cardiaque ou défibrillateur suivant la revendication 6 ou 7, **caractérisé en ce que** les électrodes (2, 4, 40, 42) consiste en une électrode bipolaire de stimulateur cardiaque ayant une pointe en carbone en tant qu'électrode de stimulation et un anneau en platine en tant qu'électrode indifférente.

**9.** Stimulateur cardiaque suivant l'une quelconque des revendications 6 à 8, qui a une section (44) de stimulateur cardiaque, **caractérisé en ce qu'**il est prévu des circuits (60) de commande de la section (44) du stimulateur cardiaque, en réponse à l'unité (54) de mesure.

FIG 1

FIG 2

FIG 3

FIG 4

FIG 5

FIG 6

FIG 7